# EUROPÄISCHE PATENTANMELDUNG

(11) **EP 2 543 411 A1**
(43) Veröffentlichungstag der Anmeldung: **09.01.2013**
(21) Anmeldenummer: 12172401.7
(22) Anmeldetag: 18.06.2012
(51) Int. Cl.: A61N 1/372

(54) **Medizinisches Implantat und Verfahren zur sicheren Implantatkommunikation**

(30) Priorität: 06.07.2011 US 201161504707 P
(71) Anmelder: BIOTRONIK SE & Co. KG, 12359 Berlin (DE)
(72) Erfinder: Diebold, Michael, 12169 Berlin (DE)
(74) Vertreter: Lindner-Vogt, Karin L.

(57) **Zusammenfassung**

Die Erfindung betrifft ein implantierbares medizinisches Gerät als aktives elektrisches Implantat mit einer Telemetrieeinheit zum drahtlosen Empfangen von Datensignalen und Steuerbefehlen, sowie mit einer Steuereinheit, die mit der Telemetrieeinheit verbunden ist und die eine Vergleichseinheit aufweist, die ausgebildet ist, über die Telemetrieeinheit empfangene Datensignale mit aktuell im implantierbaren medizinischen Gerät generierten oder vom implantierbaren medizinischen Gerät erfassten Datensignalen zu vergleichen. Die Datensignale repräsentieren jeweils spezifische Merkmalssequenzen, die miteinander verglichen werden können. Die Vergleichseinheit ist ausgebildet, für den Fall, dass über die Telemetrieeinheit empfangene Datensignale ein Merkmal, eine Merkmalskombination oder eine Merkmalssequenz repräsentieren, die einem oder einer durch aktuell im implantierbaren medizinischen Gerät generierte oder vom implantierbaren medizinischen Gerät erfasste Datensignale repräsentierten Merkmal, Merkmalskombination oder Merkmalssequenz gemäß einem vorgegebenen Ähnlichkeitsmaß ähnelt, ein Freigabesignal zu erzeugen. Die Steuereinheit ist ausgebildet, auf ein solches Freigabesignal anzusprechen und daraufhin über die Telemetrieeinheit empfangene Steuerbefehle auszuführen oder Steuerbefehle über die Telemetrieeinheit zu empfangen und andernfalls Steuerbefehle nicht auszuführen oder nicht zu empfangen.

## Beschreibung

Die Erfindung betrifft ein implantierbares medizinisches Gerät mit einer Telemetrieeinheit zum drahtlosen Empfangen von Datensignalen und Steuerbefehlen, sowie mit einer Steuereinheit, die mit der Telemetrieeinheit verbunden ist.

Ein solches implantierbares medizinisches Gerät - beispielsweise ein implantierbarer Herzschrittmacher oder ein implantierbarer Kardioverter/Defibrillator - kann über seine Telemetrieeinheit Steuerbefehle empfangen, die typischerweise die Funktionsweise des implantierbaren medizinischen Gerätes verbessern sollen, die im Missbrauchsfall aber auch die Funktionsweise beeinträchtigen können. Daher sollte eine Datenkommunikation zwischen einem externen Gerät und dem implantierbaren medizinischen Gerät möglichst sicher sein, insbesondere authentifiziert sein.

Untersuchungen haben gezeigt, dass der Zugriff von extern auf ein aktives elektrisches Implantat als ein implantierbares medizinisches Gerät nur einer autorisierten Person oder einem autorisierten externen Gerät gestattet werden darf. Dies ist notwendig in Anwendungsszenarien, an denen potenziell gesundheitsbeeinträchtigende Umkonfigurationen am Implantat gemacht werden sollen (Stichwort Fernprogrammierung von Implantaten via Home Monitoring).

Um sicherzustellen, dass die Patientensicherheit gewährleistet ist und nur eine befugte Person Umkonfigurationen vornimmt, ist heute ein induktiver Programmierkopf zum Übertragen von Daten Standard. Seine Reichweite beträgt wenige Zentimeter, so dass der umkonfigurierende Arzt sich in unmittelbarer Nähe zum Patienten befinden muss und somit die Authentizität implizit gegeben ist.

In zukünftigen Szenarien sind Anwendungen denkbar, bei denen der Arzt selbst sich weiter entfernt befindet und eine Umkonfiguration aus der Ferne ausführen möchte. Hierzu soll die induktive Telemetrie durch eine Radiofrequenztelemetrie ersetzt werden, um dem Patienten die mitunter komplizierte und für einen Laien störanfällige Platzierung des Programmierkopfes zu ersparen.

Aus der Verwendung der Radiofrequenztelemetrie mit ihrer größeren Reichweite ergeben sich neue Möglichkeiten, die Patientensicherheit zu kompromittieren. Dies wurde bereits in mehreren Veröffentlichungen demonstriert. So sind Reichweiten im Bereich einiger Meter technisch leicht zu erreichen und damit ein Zugang zum Implantat des Patienten möglich, ohne sich unmittelbar in seiner Nähe zu befinden.

Hieraus ergibt sich der Wunsch, eine Kommunikation mit dem Implantat nur dann zuzulassen, wenn das externe Gerät unmittelbar am Patienten ein Merkmal abgreift, welches nur direkt und eventuell mit seiner Zustimmung abgegriffen werden kann und vom Implantat zur Authentifizierung mit einer eigenen Messung verglichen wird. Bei Übereinstimmung kann vom Implantat dann eine Kommunikation freigegeben werden.

Es gibt eine Reihe von Vorschlägen, welche Art von Merkmalen extrahiert werden können, um eine Authentifizierung durchzuführen:
- Optische Pulsmessung und Vergleich mit einem intrakardialen Elektrokardiogramm (IEGM)
- Laufzeitmessung der Radiosignale und Verweigerung des Zugriffs bei zu großer Entfernung
- Eintätowierung eines Zugangscodes als Barcode oder Zahl, der mit einer Kamera abgelesen werden muss

Die bekannten Lösungen zeichnen sich in der Regel dadurch aus, dass sie zusätzliche Hardware benötigen oder keine vollständige Lösung der Fragestellung erlauben. Hier eine kurze Zusammenstellung des jeweiligen Implementierungsaufwands:

### Pulsmessung

Der Implementierungsaufwand ist erhöht, da ein externes Gerät mit einem optischen Pulsmesser ausgestattet werden muss. Dies geschieht meist am Finger. Die Lösung ist jedoch im Prinzip gut, da ein patientenspezifisches Merkmal abgegriffen wird und der Patient aktiv die Messung "erlauben" muss.

### Laufzeitmessung

Der Implementierungsaufwand ist hoch, da ein spezieller RF-Chip entwickelt werden muss. Eine Authentifizierung ist möglich, jedoch ist eine Freigabe durch den Patienten nicht möglich. Zudem ist das Verfahren für den Patienten schwer zu verstehen.

### Tätowierung

Es ist nicht besonders praktikabel, Merkmale zu tätowieren. Die Sicherheit der Authentifizierung ist im Prinzip gut, weil die Authentifizierung nur nach Einwilligung des Patienten möglich ist.

Es gilt also, ein Verfahren und eine dafür geeignete Vorrichtung zu finden, welches einerseits möglichst sicher und andererseits mit vertretbarem Aufwand verbunden ist.

Erfindungsgemäß wird diese Aufgabe durch ein implantierbares medizinisches Gerät als aktives elektrisches Implantat mit einer Telemetrieeinheit zum drahtlosen Empfangen von Datensignalen und Steuerbefehlen sowie mit einer Steuereinheit gelöst, die mit der Telemetrieeinheit verbunden ist und die eine Vergleichseinheit aufweist, die ausgebildet ist, über die Telemetrieeinheit empfangene Datensignale mit aktuell im implantierbaren medizinischen Gerät generierten oder vom implantierbaren medizinischen Gerät erfassten Datensignalen zu vergleichen. Die Datensignale repräsentieren jeweils spezifische Merkmalssequenzen, die miteinander verglichen werden können. Die Vergleichseinheit ist ausgebildet für den Fall, dass über die Telemetrieeinheit empfangene Datensignale ein Merkmal, eine Merkmalskombination oder eine Merkmalssequenz repräsentieren, die einem oder einer durch aktuell im implantierbaren medizinischen Gerät generierte oder vom implantierbaren medizinischen Gerät erfasste Datensignale repräsentierten Merkmal, Merkmalskombination oder Merkmalssequenz gemäß einem vorgegebenen Ähnlichkeitsmaß ähnelt, ein Freigabesignal zu erzeugen. Die Steuereinheit ist ausgebildet, auf ein solches Freigabesignal anzusprechen und daraufhin über die Telemetrieeinheit empfangene Steuerbefehle auszuführen oder Steuerbefehle über die Telemetrieeinheit zu empfangen und andernfalls Steuerbefehle nicht auszuführen oder nicht zu empfangen.

Die jeweilige durch ein Datensignal repräsentierte Merkmalssequenz kann eine Sequenz physiologischer Merkmale sein, die vom Implantat erfasst werden können oder sie kann eine Sequenz vom Implantat selbst erzeugter Signale sein. Ein Beispiel für eine Sequenz physiologischer Merkmale, die vom Implantat erfasst werden können, ist die Sequenz der Zyklusdauern aufeinanderfolgender Herzzyklen, die vom Implantat in ein Datensignal gewandelt wird, das die einzelnen Zyklusdauern als aufeinanderfolgende Merkmale und damit als Merkmalssequenz repräsentiert.

Die Erfindung schließt die Erkenntnis ein, dass beim Aufbau einer sicheren Datenkommunikation zwischen Implantat und externem Gerät zur Wahrung der Authentizität Merkmale zum Einsatz kommen müssen, die nur den Kommunikationspartnern bekannt sind. Bei dem hier vorgestellten medizinischen Gerät sind dies Merkmale, die im Gerät selbst jeweils aktuell erfasst oder generiert werden und welche daher auch von einem externen Gerät (oder einer Kombination externer Geräte) nur mit Zustimmung des Patienten abgegriffen werden können. Anstelle einer Merkmalssequenz können die zu vergleichenden Datensignale auch nur ein einziges, jeweils aktuell vom Implantat erfassbares oder generiertes Merkmal oder eine Kombination simultan aktuell erfassbarer oder aktuell generierter Merkmale repräsentieren.

Gemäß einer Ausführungsvariante, bei der das jeweils aktuell vom Implantat erfasste Datensignal eine Merkmalssequenz repräsentiert, die aus einem intrakardialen Elektrokardiogramm abgeleitet ist, werden mit einem externen Gerät, beispielsweise einem Smartphone, die Herztöne eines Patienten aufgenommen, spezifische Merkmale extrahiert und zum Abgleich an das Implantat gesendet. Das Implantat vergleicht diese spezifischen Merkmale mit einem aufgenommenen intrakardialen Elektrokardiogramm (IEGM) und lässt nur bei einer hinreichenden Übereinstimmung eine Datenkommunikation oder das Ausführen von Steuerbefehlen zu.

Gemäß weiterer Ausführungsvarianten kann das externe Gerät, ggf. in Kombination mit einem weiteren externen Gerät, dazu ausgebildet sein
a) eine Messung der Herztöne
b) eine Messung eines EKGs und/oder
c) eine Messung spezieller unterschwelliger Impulse des Implantates
durchzuführen.

Im Falle der Varianten a) und b) besitzt das implantierbare medizinische Gerät eine Erfassungseinheit zum Erfassen jeweils aktueller Herzrhythmussignale und Generieren von jeweils aktuell vom implantierbaren medizinischen Gerät erfassten Datensignalen. Die Erfassungseinheit ist mit der Steuereinheit verbunden und die Vergleichseinheit der Steuereinheit ist ausgebildet, über die Telemetrieeinheit empfangene Datensignale mit von der Erfassungseinheit erfassten aktuellen Herzrhythmussignalen zu vergleichen, und für den Fall, dass über die Telemetrieeinheit empfangene Datensignale einen Herzrhythmus repräsentieren, der einem durch jeweils aktuelle Datensignale repräsentierten aktuellem Herzrhythmus gemäß einem vorgegebenen Ähnlichkeitsmaß ähnelt, ein Freigabesignal zu erzeugen. Die Steuereinheit ist ausgebildet, auf das Freigabesignal anzusprechen und daraufhin über die Telemetrieeinheit empfangene Steuerbefehle ausführen oder Steuerbefehle über die Telemetrieeinheit zu empfangen und andernfalls Steuerbefehle nicht auszuführen oder nicht zu empfangen.

Bei dieser Ausführungsvariante werden beispielsweise mit Hilfe des externen Gerätes die Herztöne des Patienten über einen Zeitraum von wenigen Sekunden gemessen. Daraus werden patientenindividuelle Merkmale, wie RR-Intervall, mittlere Frequenz, Frequenzvariation etc., bestimmt und mit einem Zeitstempel versehen.

Im Implantat wird kontinuierlich ein Ringpuffer mit einem intrakardial gemessenen Elektrokardiogramm befüllt und ebenfalls mit einem Zeitstempel versehen.

Zum Zeitpunkt des Kommunikationsbeginns wird vom externen Gerät und Implantat ein Vergleich der Uhren durchgeführt und ein Delta bestimmt. Der Zeitstempel der Messung wird vom externen Gerät um dieses Delta korrigiert, damit im Implantat leicht ein Merkmalsvergleich ausgeführt werden kann.

Stimmen die Merkmale überein, wird vom Implantat der Zugriff auf Konfigurationen freigeschaltet.

Die Varianten a) und b) entsprechen sich weitgehend, bis auf den Unterschied, dass bei der Variante b) nicht die Herztöne aufgenommen werden, sondern ein externes Elektrokardiogramm, aus dem ebenfalls patientenspezifische Merkmale bestimmt werden.

Im Falle der Variante c) besitzt das implantierbare medizinische Gerät einen Impulsgenerator, der ausgebildet ist, unterschwellige Strom- oder Spannungsimpulse zu generieren, welche so bemessen sind, dass sie keine Stimulation von Muskelzellen bewirken, wobei der Impulsgenerator weiter ausgebildet ist, unterschwellige Strom- oder Spannungsimpulse in einer Folge zu generieren, die eine nicht erratbare Merkmalssequenz repräsentiert. Außerdem weist das implantierbare medizinische Gerät vorzugsweise einen Merkmalssequenzgenerator auf, der ausgebildet ist, eine nicht erratbare Merkmalssequenz zu generieren und der mit dem Impulsgenerator verbunden ist, wobei der Impulsgenerator ausgebildet ist, unterschwellige Strom- oder Spannungsimpulse zu generieren, die die nicht erratbare Merkmalsequenz repräsentieren.

Gemäß dieser Variante c) geschieht die Datenübertragung mit Hilfe von unterschwelligen Impulsen. Das heißt, Implantat und externes Gerät haben je einen Transmitter, die über kurze Spannungspulse unterhalb der Reizschwelle der Herzzellen Daten austauschen.

Das Implantat erzeugt eine nicht erratbare Sequenz, die es an das externe Gerät überträgt. Nur dieses externe Gerät ist in der Lage, die Sequenz des Implantates zu empfangen.

Die Telemetrieeinheit des implantierbaren medizinischen Gerätes kann in allen Fällen, vorzugsweise aber im Fall der Variante c), dazu ausgebildet sein, mit einer entsprechenden Telemetrieschnittstelle des externen Gerätes Daten über unterschwellige Strom- oder Spannungsimpulse auszutauschen.

Insbesondere bei den Varianten a) und b) ist es bevorzugt, wenn jeweils aktuell im implantierbaren medizinischen Gerät generierte oder vom implantierbaren medizinischen Gerät erfasste Datensignale Zeitstempelsignale enthalten, die einzelnen Merkmale in einer durch jeweilige aktuell im implantierbaren medizinischen Gerät generierte oder vom implantierbaren medizinischen Gerät erfasste Datensignale repräsentierten Merkmalssequenz zugeordnet sind und die den Zeitpunkt des Auftretens oder Erfassens eines Merkmals gemäß eines Geräte-internen Zeitgebers angeben. Dies erleichtert den zeitrichtigen Vergleich der vom implantierbaren medizinischen Gerät generierten oder erfassten Datensignale mit den vom externen Gerät an das implantierbare medizinische Gerät übertragenen Datensignalen, indem die zu vergleichenden Datensignale mittels der Zeitstempel synchronisiert werden können.

Die der Erfindung zugrundeliegende Aufgabe wird auch durch ein externes Gerät mit einer Telemetrieschnittstelle zum drahtlosen Übertragen von Datensignalen und Steuerbefehlen gelöst, das eine Erfassungseinheit zum Erfassen einer patientenindividuellen physiologischen oder durch ein implantierbares medizinisches Gerät generierten Merkmalssequenz aufweist und ausgebildet ist, eine jeweils aktuell erfasste Merkmalssequenz repräsentierende Datensignale über die Telemetrieschnittstelle an ein implantierbares medizinisches Gerät zu übertragen.

Gemäß der vorgenannten Varianten a) und b) ist die Erfassungseinheit als Erfassungseinheit zum Erfassen von einer den jeweils aktuellen Herzrhythmus eines Patienten repräsentierenden Merkmalssequenz ausgebildet.

Insbesondere ist die Erfassungseinheit gemäß der vorgenannten Variante a) ausgebildet, Herztöne eines Patienten als Merkmalssequenz zu erfassen und entsprechende Datensignale zu bilden.

Gemäß der vorgenannten Variante b) ist die Erfassungseinheit ausgebildet ist, ein Oberflächen-Elektrokardiogramm eines Patienten als Merkmalssequenz zu erfassen und entsprechende Datensignale zu bilden.

Gemäß der vorgenannten Variante c) ist die Erfassungseinheit eine Erfassungseinheit zum Erfassen von unterschwelligen Strom- oder Spannungsimpulsen am Körper eines Patienten.

Ein weiterer Aspekt der Erfindung betrifft ein Verfahren zum Einleiten einer sicheren Datenkommunikation mit einem implantierbaren medizinischen Gerät. Erfindungsgemäß weist dieses Verfahren die folgenden Verfahrensschritte auf:
Erfassen oder Generieren einer Merkmalssequenz oder Merkmalskombination in einem implantierbaren medizinischen Gerät.
Erfassen einer Merkmalssequenz oder Merkmalskombination mit einem externen Gerät.
Übertragen von Datensignalen, die eine von dem externen Gerät erfasste Merkmalssequenz oder Merkmalskombination repräsentieren, an das implantierbare medizinische Gerät.
Vergleichen der von dem implantierbaren medizinischen Gerät erfassten oder generierten Merkmalssequenz oder Merkmalskombination mit der durch die vom externen Gerät an das implantierbare medizinische Gerät übertragenen Datensignale repräsentierten Merkmalssequenz oder Merkmalskombination;
   und
Zulassen oder Unterbinden eines weiteren telemetrischen Datenaustauschs oder Zulassen oder Unterbinden des Ausführens vom externen Gerät an das implantierbare medizinische Gerät übertragener Steuerbefehle in Abhängigkeit des Ergebnisses des Vergleichs.

Ein telemetrischer Datenaustausch und/oder das Ausführen von Steuerbefehlen wird zugelassen, wenn der Vergleich eine hinreichende Ähnlichkeit der durch die verglichenen Datensignale repräsentierten Merkmalssequenzen oder Merkmalskombinationen ergibt.

Ein weiterer Aspekt der Erfindung betrifft ein System aus einem implantierbaren medizinischen Gerät der hier vorgestellten Art und einem oder mehreren externen Geräten.

Die Erfindung soll nun anhand eines Ausführungsbeispiels unter Bezug auf die Figuren näher erläutert werden. Diese zeigen in:
- Fig. 1: eine schematische Darstellung eines Herztherapiesystems;
- Fig. 2: eine Darstellung eines Herzstimulators mit angeschlossenen, im Herzen angeordneten Elektroden;
- Fig. 3: ein schematisches Blockschaltbild eines Herzstimulators;
- Fig. 4: ein Smartphone zum Abhören von Herztönen; und
- Fig. 5: eine Armbanduhr mit MICS-Telemetrie oder mit unterschwelliger Telemetrie.

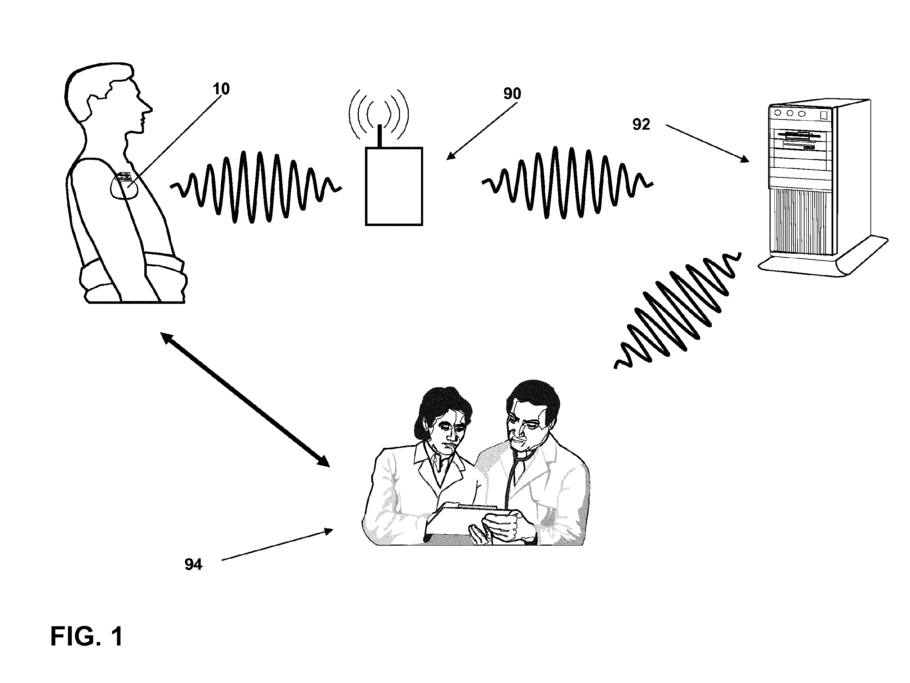

In Figur 1 ist zur Übersicht ein Herztherapiesystem dargestellt, das neben einem implantierten Herzschrittmacher 10 ein externes Gerät (Patientengerät) 90 sowie ein durch einen Server symbolisch dargestelltes Servicecenter 110 umfasst.

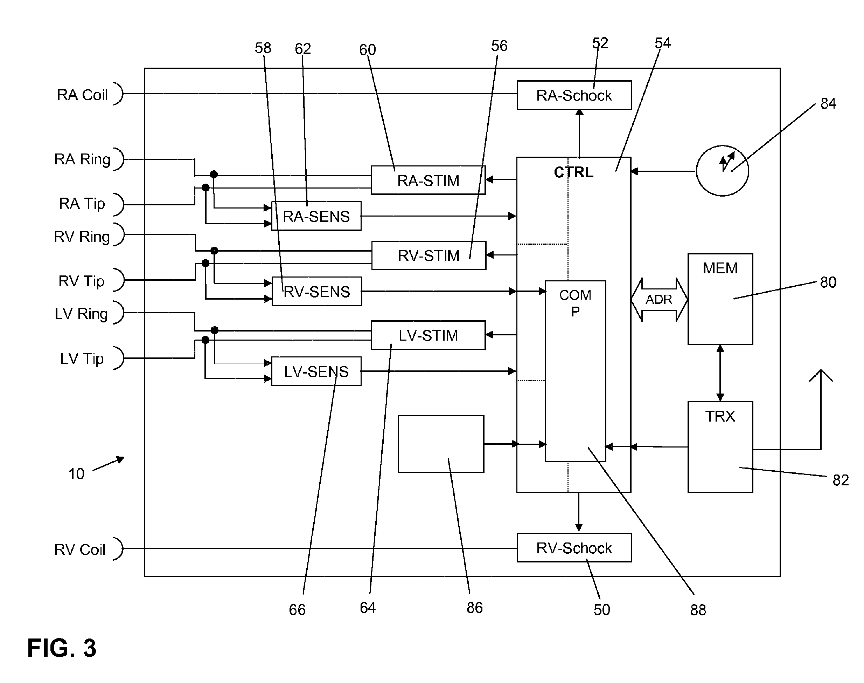

Der implantierbare Herzstimulator 10 besitzt eine Telemetrie-Einheit 82 (siehe Figur 3), über die er drahtlos Daten mit dem externen Gerät 90 austauschen kann. Das externe Gerät 90 ist beispielsweise drahtlos mit dem Servicecenter 92 verbunden, so dass insgesamt Daten zwischen dem Servicecenter 92 und dem implantierbaren Herzstimulator 10 über das externe Gerät 90 als Relaisstation ausgetauscht werden können. Ein Ärzteteam 94 kann über einen datentechnischen Zugriff auf das Servicecenter 92 Einsicht in Daten nehmen, die das Servicecenter 92 von dem implantierbaren Herzstimulator 10 erhalten hat.

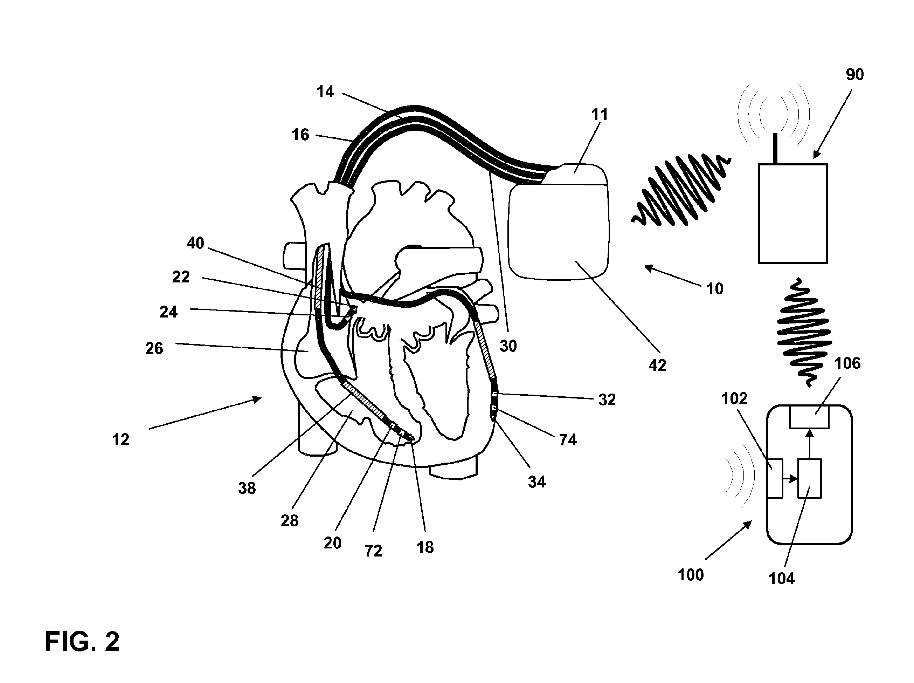

Figur 2 zeigt den implantierbaren Herzstimulator 10 in Form eines Dreikammerherzschrittmachers/Kardioverters/Defibrillators mit daran angeschlossenen Elektrodenleitungen 14, 16 und 30 in Verbindung mit einem Herz 12. Außerdem ist noch einmal das externe Gerät 90 in der Nähe des implantierbaren Herzstimulators 10 dargestellt. Die Elektrodenleitungen 14, 16 und 30 sind über bekannte standardisierte Steckverbindungen mit Kontaktbuchsen in einem Header (Anschlussgehäuse) 11 des Herzstimulators 10 elektrisch verbunden. Auf diese Weise sind die Elektrodenleitungen 14, 16 und 30 auch mit elektronischen Komponenten im Inneren eines hermetisch dichten Metallgehäuses 42 des Herzstimulators 10 verbunden. Diese Komponenten sind nachfolgend detaillierter schematisch dargestellt und bestimmen die erfindungsgemäße Funktionsweise des Herzstimulators 10.

Der Herzstimulator 10 ist ein implantierbares medizinisches Gerät und wird nachfolgend deshalb auch kurz als Implantat bezeichnet.

Die Elektrodenleitung 14 ist eine rechtsatriale Elektrodenleitung und besitzt an ihrem distalen Ende eine atriale Spitzenelektrode RA Tip 22 sowie in kurzer Entfernung davon eine atriale Ringelektrode RA Ring 24, die beide im rechten Atrium 26 des Herzens 12 platziert sind.

Die Elektrodenleitung 16 ist eine rechtsventrikuläre Elektrodenleitung und besitzt an ihrem distalen Ende eine rechtsventrikuläre Spitzenelektrode RV Tip 18 und in unmittelbarer Nähe eine rechtsventrikuläre Ringelektrode RV Ring 20. Beide Elektroden sind im Apex des rechten Ventrikels 28 des Herzens 12 angeordnet.

Außerdem ist die rechtsventrikuläre Elektrodenleitung 16 noch eine rechtsventrikuläre Schockwendel RV Schock 38 als großflächige Elektrode zur Abgabe von Relationsschocks. Eine weitere Schockwendel 40 ist in der Vena cava superior angeordnet und wird deshalb nachfolgend auch als SVC-Schockelektrode bezeichnet.

Die Elektrodenleitung 30 ist eine linksventrikuläre Elektrodenleitung, an deren distalem Ende eine linksventrikuläre Spitzenelektrode LV Tip 34 sowie in deren Nähe eine linksventrikuläre Ringelektrode LV Ring 32 angeordnet ist. Außerdem trägt die linksventrikuläre Elektrodenleitung 30 eine nicht näher bezeichnete, aber in Figur 2 dargestellte linksventrikuläre Schockwendel zur Abgabe von Defibrillationsschocks an den linken Ventrikel. Die linksventrikuläre Elektrodenleitung 30 wird vom rechten Atrium 26 des Herzens 12 aus über den Koronarsinus in eine von diesem abzweigende Lateralvene geführt und wird deswegen auch als Koronarsinus-Elektrodenleitung oder CS-Elektrodenleitung bezeichnet.

Figur 2 zeigt außerdem zwei externe Geräte 90 und 100, die zusammen eine Kombination externer Geräte bilden.

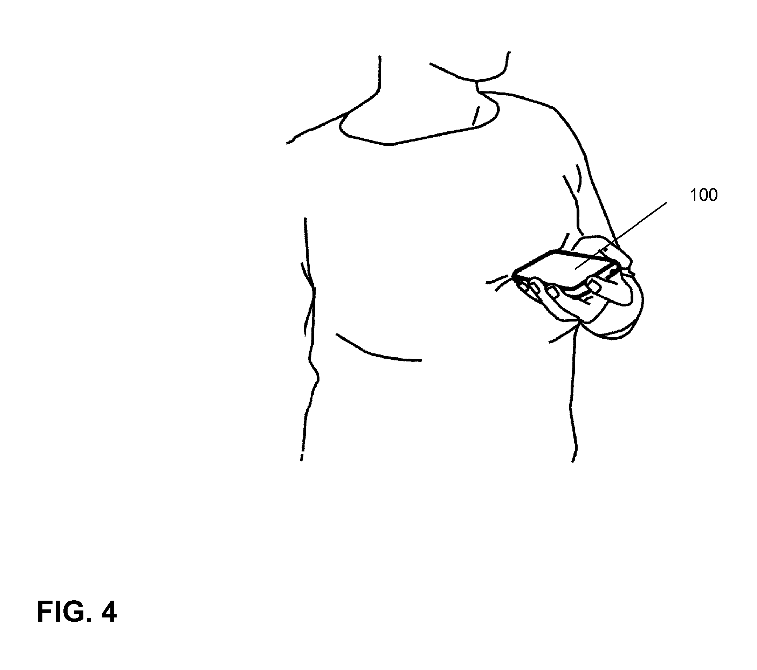

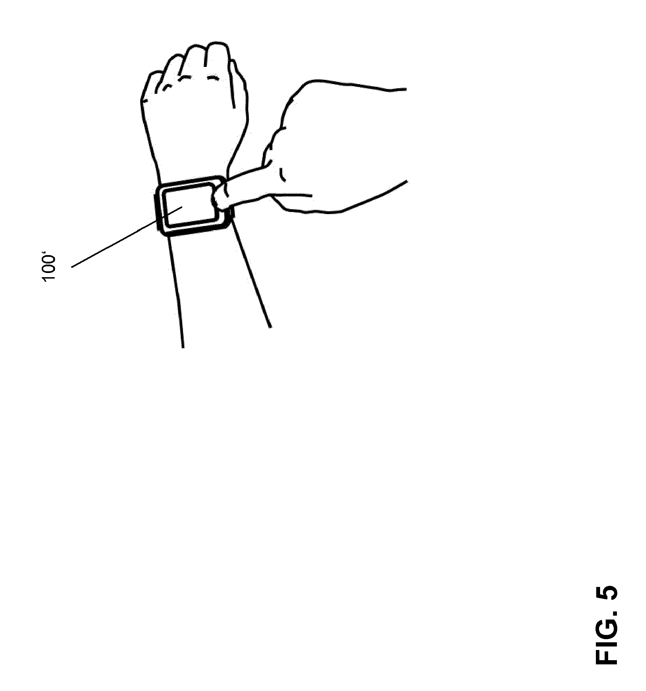

Von diesen Geräten ist das externe Gerät 90 dazu ausgebildet, Daten mit dem implantierbaren Herzstimulator 10 auszutauschen. Das externe Gerät 10 kann auf diesem Wege auch Steuer- und Programmierbefehle an die Telemetrieeinheit 82 des implantierbaren Herzstimulators 10 übertragen. Das externe Gerät 100 dient zum Erfassen einer aktuellen Patienten-spezifischen Merkmalssequenz. Dazu weist das zweite externe Gerät 100 eine Erfassungseinheit 102 auf, die beispielsweise ein Mikrofon enthält und dazu ausgebildet ist, Herztöne eines Patienten zu erfassen oder die ausgebildet ist, ein Oberflächenelektrokardiogramm über die Haut eines Patienten aufzunehmen oder die dazu ausgebildet ist, unterschwellige, vom implantierbaren Herzstimulator 10 generierte Strom- oder Spannungsimpulse zu erfassen. In einer Verarbeitungseinheit 104 werden die vom zweiten externen Gerät 100 erfassten Merkmalssequenzen weiterverarbeitet und einer Sendeeinheit 106 zugeführt, die ein eine jeweilige Merkmalssequenz repräsentierendes Datensignal an das erste externe Gerät 90 übermittelt. Damit ist dieses erste externe Gerät 90 in der Lage, das von dem zweiten externen Gerät 100 generierte Datensignal zum implantierbaren Herzstimulator 10 zu übertragen, um sich auf diese Weise zu authentifizieren. Das zweite externe Gerät 100 kann beispielsweise ein mobiles Telefon (beispielsweise ein Smartphone) sein oder nach Art einer Armbanduhr gestaltet sein. Beispiele hierfür finden sich in den Figuren 4 und 5.

Anstelle zweier externer Geräte 90 und 100 kann die Erfassungseinheit 102 auch integraler Bestandteil des ersten externen Gerätes 90 sein. Die Funktionsweise ist dann analog, wobei eine Datenverbindung zwischen zwei externen Geräten dann obsolet ist.

In Figur 3 sind die Hauptbestandteile des Herzstimulators 10 dargestellt. Auf der linken Seite sind die elektrischen Anschlüsse für die verschiedenen Elektroden 18, 20, 22, 24, 32, 34, 38 und 40 dargestellt. Die Schockelektroden 38 und 40 sind jeweils mit einem rechtsventrikulären Schockimpulsgenerator 50 bzw. SVC-Schockgenerator 52 verbunden. Beide Schockgeneratoren 50 und 52 sind mit einer Steuereinheit 54 verbunden, die die beiden Schockimpulsgeneratoren 50 und 52 bei Bedarf zur Erzeugung und Abgabe eines Defibrillationsschocks ansteuert.

Der Anschluss für die rechtsventrikuläre Tippelektrode RV Tip sowie der Anschluss für die rechtsventrikuläre Ringelektrode RV Ring sind jeweils sowohl mit einer rechtsventrikulären Stimulationseinheit 56 als auch mit einer rechtsventrikulären Sensing-Einheit 58 verbunden. Sowohl die rechtsventrikuläre Stimulationseinheit 56 als auch die rechtsventrikuläre Sensing-Einheit 58 sind jeweils mit der Steuereinheit 54 verbunden.

Die rechtsventrikuläre Stimulationseinheit 56 ist dazu ausgebildet, auf ein Ansteuersignal der Steuereinheit 54 hin einen rechtsventrikulären Stimulationsimpuls zu erzeugen und im Anschluss über die rechtsventrikuläre Ringelektrode und die rechtsventrikuläre Tippelektrode abzugeben. Alternativ ist es auch möglich, dass das Gehäuse 42 des Herzstimulators 10 eine neutrale Elektrode bildet und die rechtsventrikuläre Stimulationseinheit 56 mit dem Anschluss für die rechtsventrikuläre Ringelektrode RV Tip und dem Gehäuse 42 als andere Elektrode zur Abgabe eines Stimulationsimpulses verbunden ist. Ein rechtsventrikulärer Stimulationsimpuls unterscheidet sich von einem Defibrillationsschock dadurch, dass der Stimulationsimpuls eine wesentlich geringere Impulsstärke besitzt, so dass er nicht wie ein Defibrillationsschock auf einen Schlag das vollständige Herzgewebe (Myokard) einer Herzkammer erregt, sondern nur die Herzmuskelzellen in unmittelbarer Umgebung der rechtsventrikulären Spitzenelektrode 18. Diese Erregung breitet sich dann durch natürliche Reizleitung über den gesamten Ventrikel weiter aus und sorgt so für eine stimulierte Kontraktion des Ventrikels.

Die rechtsventrikuläre Sensing-Einheit 58 ist dazu ausgebildet, an dem Anschluss für die rechtsventrikuläre Ringelektrode RV Ring und die rechtsventrikuläre Tippelektrode RV Tip anliegende elektrische Potentiale zunächst durch einen Eingangsverstärker zu verstärken und zu filtern. Weiterhin ist die rechtsventrikuläre Sensing-Einheit ausgebildet, den Verlauf der an ihren Eingängen anliegenden elektrischen Signale derart auszuwerten, dass die rechtsventrikuläre Sensing-Einheit 58 selbsttätig eine natürliche (intrinsische), d.h. selbsttätige Kontraktion des rechten Ventrikels detektiert. Dies kann beispielsweise dadurch geschehen, dass der Verlauf des an den Eingängen der rechtsventrikulären Sensing-Einheit 58 anliegenden Signals mit einem Schwellwert verglichen wird. Typischerweise ist die größte Amplitude des Signals in Form der sogenannten R-Zacke kennzeichnend für eine natürliche Kontraktion des rechten Ventrikels, die durch Schwellwertvergleich detektiert werden kann. Die rechtsventrikuläre Sensing-Einheit 58 gibt daraufhin ein entsprechendes, eine natürliche Kontraktion des rechten Ventrikels anzeigendes Ausgangssignal an die Steuereinheit 54 aus.

Die Steuereinheit 54 bildet aus den Ausgangssignalen der Sensing-Einheit 58 ein Datensignal und führt dies einer Vergleichseinheit 88 zu.

Eine R-Zacke kann auch von einem externen Gerät, beispielsweise über ein Oberflächen-Elektrokardiogramm, erfasst werden. Eine jeweilige Folge von R-Zacken repräsentiert den Herzrhythmus eines Patienten und ist als solche eine Merkmalssequenz, deren zeitliche Eigenschaften, wie die Länge der Intervalle aufeinanderfolgender Herzzyklen, für eine individuelle Person zu einem spezifischen Zeitpunkt charakteristisch sind.

Das von der rechtsventrikulären Sensing-Einheit 58 ausgegebene Signal oder daraus abgeleitete Signalfolgen bilden somit ein Datensignal, das eine für einen Augenblick und eine Person jeweils charakteristische Merkmalssequenz repräsentiert.

Die gleiche Merkmalssequenz kann das externe Gerät beispielsweise durch Aufnahme eines Oberflächen-Elektrokardiogramms oder durch Aufnahmen der Herztöne erfassen, daraus ein entsprechendes Datensignal generieren und telemetrisch an den implantierbaren Herzstimulator 10 übertragen.

Die Vergleichseinheit 88 ist ausgebildet, ein aus dem Ausgangssignal der Sensing-Einheit 58 abgeleitetes erfasstes Datensignal mit einem über eine Telemetrieeinheit 82 empfangenen Datensignal zu vergleichen.

Der Anschluss für die rechtsatriale Tippelektrode und der Anschluss für die rechtsatriale Ringelektrode sind mit einer rechtsatrialen Stimulationseinheit 60 beziehungsweise mit einer rechtsatrialen Sensing-Einheit 62 verbunden, die jeweils ihrerseits wiederum mit der Steuereinheit 54 verbunden sind. Die rechtsatriale Stimulationseinheit 60 ist dazu ausgebildet, Stimulationsimpulse zu erzeugen, deren Stärke ausreicht, das rechtsatriale Myokard zu erregen. Die rechtsatriale Sensing-Einheit 62 ist ausgebildet, aus dem Verlauf des an ihren Eingängen anliegenden Differenzsignals eine sogenannte P-Welle zu detektieren, die eine natürliche (intrinsische) Kontraktion des rechten Atriums kennzeichnet. Detektiert die rechtsatriale Sensing-Einheit 62 eine entsprechende P-Welle, erzeugt sie ein Ausgangssignal und gibt dieses an die Steuereinheit 54 weiter, welches eine natürliche Kontraktion des rechten Atriums kennzeichnet. Eine solche P-Welle kann auch von einem externen Gerät, beispielsweise über ein Oberflächen-Elektrokardiogramm, erfasst werden.

In gleicher Weise sind auch der Anschluss für die linksventrikuläre Tippelektrode LV Tip und der Anschluss für die linksventrikuläre Ringelektrode LV Ring mit einer linksventrikulären Stimulationseinheit 64 und einer linksventrikulären Sensing-Einheit 66 verbunden. Die linksventrikuläre Stimulationseinheit 64 und die linksventrikuläre Sensing-Einheit 66 sind ebenso mit der Steuereinheit 54 verbunden. Beide funktionieren ähnlich wie die bereits beschriebenen Stimulationseinheiten 56 und 60 und Sensing-Einheiten 58 und 62.

Weiterhin umfasst der Herzstimulator 10 eine Speichereinheit 80, die mit der Steuereinheit 54 verbunden ist und es erlaubt, von der Steuereinheit 54 erzeugte oder ausgewertete Signale, insbesondere einen individuellen Herzrhythmus repräsentierende Datensignale, zu speichern. Andererseits erlaubt es die Speichereinheit 80, Steuerprogramme oder Steuerbefehle für die Steuereinheit 54 in veränderbarer Form zu speichern.

Des Weiteren ist die Steuereinheit 54 mit einem Zeitgeber 84 verbunden, der es der Steuereinheit 54 unter anderem ermöglicht, Zeitpunkte und Zeitintervalle zu bestimmen, der es aber insbesondere auch ermöglicht, erfasste intrinsische Ereignisse (also erfasste P-Wellen oder R-Zacken oder beides) mit einem Zeitstempel zu versehen, so dass die von dem Herzstimulator erfassten Datensignale entsprechende Zeitstempel enthalten.

Die Speichereinheit 80 ist mit der Telemetrie-Einheit 82 verbunden, die es erlaubt, in der Speichereinheit 80 abgelegte Daten drahtlos an das externe Gerät 90 zu übertragen oder Programmier- oder Steuerbefehle seitens des externen Geräts 90 zu dem Herzstimulator 10 zu übertragen und in der Speichereinheit 80 zu speichern. Die Telemetrieeinheit 82 ist beispielsweise zu einer Datenübertragung im MICS-Band ausgebildet. Das MICS-Band ist ein Frequenzband zwischen 402 und 405 MHz, das speziell für die Datenkommunikation mit medizinischen Implantaten vorgesehen ist (MICS: Medical Implant Communication Service). Über die Telemetrieeinheit 82 empfängt der Herzstimulator zu Beginn einer Übertragung insbesondere von Steuerbefehlen ein Datensignal, welches das sendende externe Gerät aus einer am Körper eines Patienten aufgenommen Merkmalssequenz gewonnen hat. Das empfangene Datensignal enthält ebenfalls Zeitstempel zu den durch das Datensignal repräsentierten Ereignissen.

Dies erlaubt es der Steuereinheit 54 und deren Vergleichseinheit 88, ein jeweils von ihr selbst erfasstes Datensignal mit einem seitens eines externen Gerätes empfangenen Datensignals zu vergleichen. Ergibt der Vergleich, dass das empfangene Datensignal eine Merkmalssequenz repräsentiert, die innerhalb vorgegebener Toleranzen der von dem erfassten Datensignal repräsentierten Merkmalssequenz entspricht, gibt die Steuereinheit 54 eine weitere Datenkommunikation oder das Ausführen seitens des externen Gerätes 90 empfangener Steuerbefehle frei, andernfalls unterbindet sie eine weitere Datenkommunikation oder das Ausführen der Steuerbefehle.

In dem soweit geschilderten Ausführungsbeispiel generiert das Herz des jeweiligen Patienten eine nicht erratbare Merkmalssequenz, die sowohl von dem Herzstimulator 10 als auch dem externen Gerät 100 erfasst und jeweils in ein Datensignal umgesetzt werden kann.

Alternativ kann der Herzstimulator 10 jedoch auch einen Merkmalssequenzgenerator 86 aufweisen, der der ausgebildet ist, eine nicht erratbare Merkmalssequenz zu generieren. Der Merkmalssequenzgenerator 86 ist mit wenigstens einer der Stimulationseinheiten 56, 60 oder 64 direkt oder indirekt, beispielsweise über die Steuereinheit 54, verbunden und veranlasst diese, unterschwellige Strom- oder Spannungsimpulse in einer Folge zu generieren, die die nicht erratbare Merkmalsequenz repräsentiert. Eine solche Folge unterschwelliger Strom- oder Spannungsimpulse kann von dem externen Gerät 100 am Körper eines Patienten erfasst und in ein entsprechendes Datensignal umgesetzt werden.

Der Merkmalssequenzgenerator 86 ist außerdem mit der Vergleichseinheit 88 verbunden, so dass diese eine aktuelle, von dem Merkmalssequenzgenerator 86 generierte Merkmalssequenz mit einer Merkmalssequenz vergleichen kann, die durch ein seitens des externen Gerätes 90 empfangenes Datensignal repräsentiert ist. Ergibt der Vergleich, dass das empfangene Datensignal eine Merkmalssequenz repräsentiert, die innerhalb vorgegebener Toleranzen der von dem Merkmalssequenzgenerator 86 generierten Merkmalssequenz entspricht, gibt die Steuereinheit 54 eine weitere Datenkommunikation oder das Ausführen seitens des externen Gerätes 90 empfangener Steuerbefehle frei, andernfalls unterbindet sie eine weitere Datenkommunikation oder das Ausführen der Steuerbefehle.

In Bezug auf das in Figur 3 abgebildete Ausführungsbeispiel ist anzumerken, dass es ausreicht, wenn die Vergleichseinheit 88 entweder nur mit der Sensingeinheit 58 und der Telemetrieeinheit 82 bzw. dem Speicher 80 verbunden ist oder nur mit dem Merkmalssequenzgenerator 86 und der Telemetrieeinheit 82 bzw. dem Speicher 80.

Figur 4 zeigt ein zweites externes Gerät 100 in Form eines Smartphones zum Abhören von Herztönen, das ein die abgehörten Herztöne repräsentierendes Signal via Bluetooth Low Energy an das externe Gerät 90 oder alternativ direkt an den Herzstimulator 10 übertragen kann.

Im Falle der letztgenannten Variante kommt als Kommunikationstechnologie zwischen dem Herzstimulator 10 und Smartphone Bluetooth Low Energy oder eine vergleichbare, im Smartphone verfügbare RF- oder NF-Kommunikationstechnologie zum Einsatz.

Zum Einleiten einer sicheren Datenkommunikation mit dem Herzstimulator 10 hält ein Patient das externe Gerät 100 mit dem Mikrofon 102 an seine Brust, um eine Folge von Herztönen aufzunehmen. Zeitgleich findet eine Aufzeichnung der elektrischen Signale des Herzens im Ringpuffer als Teil das Speichers 80 des Implantates 10 statt. Beide Aufzeichnungen sind mit einem Zeitstempel versehen. Zu Beginn der nun folgenden Kommunikationssequenz stellen Implantat 10 und externes Gerät 90 oder 100 die Differenz ihrer internen Uhren fest, um eine zeitliche Korrelation der beiden Aufnahmen sicherstellen zu können.

Das externe Gerät 100 extrahiert aus den Herztönen den zeitlichen Abstand mehrerer aufeinanderfolgender Herzschläge, die Patienten-individuell sind. Diese Information wird an das Implantat 10 übertragen und dort mit den aus gespeicherten intrakardialen Elektrokardiogrammen gewonnenen Merkmalen verglichen. Stellt die Vergleichseinheit 88 des Implantats 10 eine Korrelation fest, gibt es den Zugriff auf die Konfiguration frei.

Figur 5 zeigt ein zweites externes Gerät 100' in Form einer Armbanduhr mit MICS-Telemetrie oder mit unterschwelliger Telemetrie.

Gemäß einer ersten Untervariante b) dieser Ausführungsvariante wird eine Armbanduhr mit MICS-Telemetrie genutzt. Der Patient löst durch die Berührung der Lünette eine Aufzeichnung des zwischen beiden Händen gemessenen EKGs aus. Ebenso wie in der beschriebenen Ausführungsvariante geschieht eine Speicherung mit Zeitstempel und Merkmalsextraktion.

Gemäß einer alternativen Untervariante c) wird eine Armbanduhr mit unterschwelliger Telemetrie genutzt.

Der Patient berührt dazu die Lünette einer mit dieser Technologie ausgestatteten Armbanduhr, die als Elektrode ausgebildet ist. Die zweite Elektrode bildet der Gehäuseboden auf der Unterseite der Uhr.

Das Implantat 10 erzeugt eine nicht erratbare Sequenz, z.B. abgeleitet durch Digitalisierung eines verrauschten Signals, um eine bestmögliche Zufälligkeit zu erreichen, die es an das externe Gerät überträgt. Nur dieses externe Gerät ist in der Lage, die Sequenz des Implantates über Lünette und Gehäuseboden zu empfangen.

Die verschiedenen varianten bieten die folgenden Vorteile:

Die Lösung gemäß Variante a) zeichnet sich dadurch aus, dass als externes Gerät heute im Markt weit verbreitete Smartphones verwendet werden können. Somit beschränkt sich die Entwicklung auf eine entsprechende Software für verschiedene Endgeräteplattformen unter der Annahme, dass zukünftige Implantate eine gebräuchliche RF-Telemetrie (z.B.: Bluetooth Low Energy) verwenden. Die Lösung ist damit von den Kosten her nicht mehr von der Anzahl der Anwender abhängig.

Die Lösung gemäß Variante b) hat den Vorteil, weiterhin die bekannte MICS-Telemetrie zu verwenden und mit einem einfachen Ansatz eine zweifelsfreie Authentifizierung des Zugangs zu ermöglichen unter Einholung der Einwilligung des Patienten.

Die Lösung gemäß Variante c) hat den Vorteil, dass der Merkmalsvergleich in dieser Variante besonders einfach ist, da das Implantat eine eindeutige und nur einmal verwendete Signalfolge berechnet und als unterschwellige Impulse abgibt. Zudem ist der Stromverbrauch durch diese Art der Datenübertragung besonders niedrig.

## Patentansprüche

1. Implantierbares medizinisches Gerät mit einer Telemetrieeinheit zum drahtlosen Empfangen von Datensignalen und Steuerbefehlen,
sowie mit einer Steuereinheit, die mit der Telemetrieeinheit verbunden ist und eine Vergleichseinheit aufweist, die ausgebildet ist, über die Telemetrieeinheit empfangene Datensignale mit aktuell im implantierbaren medizinischen Gerät generierten oder vom implantierbaren medizinischen Gerät erfassten Datensignalen zu vergleichen, und für den Fall, dass über die Telemetrieeinheit empfangene Datensignale ein Merkmal, eine Merkmalssequenz oder eine Merkmalskombination repräsentieren, die einem oder einer durch aktuell im implantierbaren medizinischen Gerät generierte oder vom implantierbaren medizinischen Gerät erfasste Datensignale repräsentierten Merkmal, Merkmalssequenz oder Merkmalskombination gemäß einem vorgegebenen Ähnlichkeitsmaß ähnelt, ein Freigabesignal zu erzeugen, wobei die Steuereinheit ausgebildet ist, auf das Freigabesignal anzusprechen und daraufhin über die Telemetrieeinheit empfangene Steuerbefehle auszuführen oder Steuerbefehle über die Telemetrieeinheit zu empfangen und andernfalls Steuerbefehle nicht auszuführen oder nicht zu empfangen.

2. Implantierbares medizinisches Gerät nach Anspruch 1, **gekennzeichnet durch** eine Erfassungseinheit zum Erfassen jeweils aktueller Herzrhythmussignale und Generieren von jeweils aktuell vom implantierbaren medizinischen Gerät erfassten Datensignalen, wobei die Erfassungseinheit mit der Steuereinheit verbunden ist und die Steuereinheit ausgebildet ist, über die Telemetrieeinheit empfangene Datensignale mit von der Erfassungseinheit erfassten aktuellen Herzrhythmussignalen zu vergleichen, und für den Fall, dass über die Telemetrieeinheit empfangene Datensignale einen Herzrhythmus repräsentieren, der einem **durch** jeweils aktuelle Datensignale repräsentierten aktuellem Herzrhythmus gemäß einem vorgegebenen Ähnlichkeitsmaß ähnelt, ein Freigabesignal zu erzeugen, wobei die Steuereinheit ausgebildet ist, auf das Freigabesignal anzusprechen und daraufhin über die Telemetrieeinheit empfangene Steuerbefehle ausführen oder Steuerbefehle über die Telemetrieeinheit zu empfangen und andernfalls Steuerbefehle nicht auszuführen oder nicht zu empfangen.

3. Implantierbares medizinisches Gerät nach Anspruch 1, **gekennzeichnet durch** einen Impulsgenerator, der ausgebildet ist, unterschwellige Strom- oder Spannungsimpulse zu generieren, welche so bemessen sind, dass sie keine Stimulation von Muskelzellen bewirken, wobei der Impulsgenerator weiter ausgebildet ist, unterschwellige Strom- oder Spannungsimpulse in einer Folge zu generieren, die eine nicht erratbare Merkmalssequenz repräsentiert.

4. Implantierbares medizinisches Gerät nach Anspruch 3, **dadurch gekennzeichnet, dass** das implantierbare medizinische Gerät einen Merkmalssequenzgenerator aufweist, der ausgebildet ist, eine nicht erratbare Merkmalssequenz oder Merkmalskombination zu generieren und der mit dem Impulsgenerator verbunden ist, wobei der Impulsgenerator ausgebildet ist, unterschwellige Strom- oder Spannungsimpulse zu generieren, die die nicht erratbare Merkmalsequenz repräsentieren.

5. Implantierbares medizinisches Gerät nach einem der Ansprüche 1 bis 4, **dadurch gekennzeichnet, dass** jeweils aktuell im implantierbaren medizinischen Gerät generierte oder vom implantierbaren medizinischen Gerät erfasste Datensignale Zeitstempelsignale enthalten, die einzelnen Merkmale in einer durch jeweilige aktuell im implantierbaren medizinischen Gerät generierte oder vom implantierbaren medizinischen Gerät erfasste Datensignale repräsentierten Merkmalssequenz zugeordnet sind und die den Zeitpunkt des Auftretens oder Erfassens eines Merkmals gemäß eines Geräte-internen Zeitgebers angeben.

6. Implantierbares medizinisches Gerät nach einem der Ansprüche 1 bis 5, **dadurch gekennzeichnet, dass** das implantierbare medizinische Gerät ein implantierbarer Herzstimulator ist.

7. Externes Gerät mit einer Telemetrieschnittstelle zum drahtlosen Übertragen von Datensignalen und Steuerbefehlen, **dadurch gekennzeichnet, dass** das externe Gerät eine Erfassungseinheit zum Erfassen einer Patienten-individuellen physiologischen oder durch ein implantierbares medizinisches Gerät generierten Merkmalssequenz aufweist und ausgebildet ist, eine jeweils aktuell erfasste Merkmalssequenz repräsentierende Datensignale über die Telemetrieschnittstelle an ein implantierbares medizinisches Gerät zu übertragen.

8. Externes Gerät nach Anspruch 7, **dadurch gekennzeichnet, dass** die Erfassungseinheit eine Erfassungseinheit zum Erfassen von einer einen jeweils aktuellen Herzrhythmus eines Patienten repräsentierenden Merkmalssequenz ist.

9. Externes Gerät nach Anspruch 8, **dadurch gekennzeichnet, dass** die Erfassungseinheit ausgebildet ist, Herztöne eines Patienten als Merkmalssequenz zu erfassen und entsprechende Datensignale zu bilden.

10. Externes Gerät nach Anspruch 8, **dadurch gekennzeichnet, dass** die Erfassungseinheit ausgebildet ist, ein Oberflächen-Elektrokardiogramm eines Patienten als Merkmalssequenz zu erfassen und entsprechende Datensignale zu bilden.

11. Externes Gerät nach Anspruch 7, **dadurch gekennzeichnet, dass** die Erfassungseinheit eine Erfassungseinheit zum Erfassen von unterschwelligen Strom- oder Spannungsimpulsen am Körper eines Patienten ist.

12. Verfahren zum Einleiten einer sicheren Datenkommunikation zwischen einem implantierbaren medizinischen Gerät und einem externen Gerät, mit den Verfahrensschritten:
Erfassen oder Generieren einer Merkmalssequenz oder Merkmalskombination in einem implantierbaren medizinischen Gerät;
Erfassen einer Merkmalssequenz oder Merkmalskombination mit einem externen Gerät;
Übertragen von Datensignalen, die eine von dem externen Gerät erfasste Merkmalssequenz oder Merkmalskombination repräsentieren, an das implantierbare medizinische Gerät;
Vergleichen der von dem implantierbaren medizinischen Gerät erfassten oder generierten Merkmalssequenz oder Merkmalskombination mit der durch die vom externen Gerät an das implantierbare medizinische Gerät übertragenen Datensignale repräsentierten Merkmalssequenz oder Merkmalskombination; und
Zulassen oder Unterbinden eines weiteren telemetrischen Datenaustauschs oder Zulassen oder Unterbinden des Ausführens vom externen Gerät an das implantierbare medizinische Gerät übertragener Steuerbefehle in Abhängigkeit des Ergebnisses des Vergleichs.

13. System mit einem implantierbaren medizinischen Gerät gemäß einem der Ansprüche 1 bis 6 und einem externen Gerät gemäß einem der Ansprüche 7 bis 11, **dadurch gekennzeichnet, dass** die Telemetrieeinheit des implantierbaren medizinischen Gerätes und die Telemetrieschnittstelle des externen Gerätes ausgebildet sind, eine Datenkommunikation im MICS-Frequenzband durchzuführen.

14. System mit einem implantierbaren medizinischen Gerät gemäß einem der Ansprüche 1 bis 6 und einem externen Gerät gemäß einem der Ansprüche 7 bis 11, **dadurch gekennzeichnet, dass** die Telemetrieeinheit des implantierbaren medizinischen Gerätes und die Telemetrieschnittstelle des externen Gerätes ausgebildet sind, eine Datenkommunikation mittels unterschwelliger Strom- oder Spannungsimpulse durchzuführen.

15. System nach Anspruch 13 oder 14, **dadurch gekennzeichnet, dass** das System ein zweites externes Gerät aufweist, dass eine Erfassungseinheit gemäß einem der Ansprüche 8 bis 11 aufweist.
